# EUROPÄISCHE PATENTSCHRIFT

(11) **EP 2 925 381 B1**
(45) Veröffentlichungstag und Bekanntmachung des Hinweises auf die Patenterteilung: **03.01.2018**
(21) Anmeldenummer: 13799268.1
(22) Anmeldetag: 28.11.2013
(51) Int. Cl.: A61M 1/14

(54) **VORRICHTUNG UND VERFAHREN ZUR AUTORISIERUNG DER BEDIENUNG EINES MEDIZINISCHEN GERÄTS**
DEVICE AND METHOD FOR AUTHORIZING THE OPERATION OF A MEDICAL DEVICE
DISPOSITIF ET PROCÉDÉ D'AUTORISATION DE L'UTILISATION D'UN APPAREIL MÉDICAL

(30) Priorität: 28.11.2012 DE 102012111523; 28.11.2012 US 201261730546 P
(43) Veröffentlichungstag der Anmeldung: 07.10.2015
(73) Patentinhaber: Fresenius Medical Care Deutschland GmbH, 61352 Bad Homburg (DE)
(72) Erfinder: MARTERSTOCK, Stefan, Konrad, 97776 Eussenheim-Hundsbach (DE)
(74) Vertreter: Nordmeyer, Philipp Werner
(86) Internationale Anmeldenummer: PCT/EP2013/074963
(87) Internationale Veröffentlichungsnummer: WO 2014/083101

(56) Entgegenhaltungen:
- EP-A2- 1 576 972
- WO-A1-2007/061368
- WO-A1-2010/044088
- DE-A1-102010 041 338
- US-A1- 2004 172 222
- US-A1- 2007 201 025
- US-A1- 2012 075 061

## Beschreibung

### Technisches Gebiet

Die Erfindung betrifft ein medizinisches Gerät zur Behandlung eines Patienten, eine Identifikationsvorrichtung für einen Bediener eines medizinischen Geräts, ein Behandlungssystem für einen Patienten umfassend ein medizinisches Gerät sowie eine Identifikationsvorrichtung, sowie ein Verfahren zur Überprüfung einer Autorisierung eines Bedieners eines medizinischen Geräts. Das genannte medizinische Gerät beziehungsweise das Behandlungssystem dienen bevorzugt zur Durchführung einer Dialysebehandlung eines Patienten.

### Stand der Technik

Unter medizinischen Geräten werden hier insbesondere Geräte zur Leitung, Behandlung und/oder Verteilung von Flüssigkeiten und/oder Gasen verstanden, bei denen über eine Fluidleitung Fluid zwischen einem Patienten und einer Fluidbehandlungskomponente und/oder einer Fluidquelle transportiert werden.

Unter medizinischen Geräten werden insbesondere auch Fluidbehandlungsgeräte wie beispielsweise Blutbehandlungsgeräte verstanden, bei denen ein Fluid eines Patienten über eine Fluidleitung einer Fluidbehandlungskomponente zugeführt, durch die Fluidbehandlungskomponente behandelt und über die Fluidleitung, die in einen arteriellen und einen venösen Zweig aufgeteilt werden kann, wieder an den Patienten zurückgegeben wird. Beispiele für solche Blutbehandlungsgeräte sind insbesondere Hämodialysegeräte.

Ein solches Blutbehandlungsgerät ist Gegenstand der DE 198 49 787 C1 der Anmelderin.

Die Dialyse ist ein Verfahren zur Blutreinigung von Patienten mit akuter oder chronischer Niereninsuffizienz. Grundsätzlich unterscheidet man hierbei Verfahren mit einem extrakorporalen Blutkreislauf, wie die Hämodialyse, die Hämofiltration oder die Hämodiafiltration und der Peritonealdialyse, die keinen extrakorporalen Blutkreislauf aufweist.

Das Blut wird bei der Hämodialyse in einem extrakorporalen Kreislauf durch die Blutkammer eines Dialysators geleitet, die über eine semipermeable Membran von einer Dialysierflüssigkeitskammer getrennt ist. Die Dialysierflüssigkeitskammer wird von einer die Blutelektrolyte in einer bestimmten Konzentration enthaltenen Dialysierflüssigkeit durchströmt. Die Stoffkonzentration der Blutelektrolyte in der Dialysierflüssigkeit entspricht dabei der Konzentration im Blut eines Gesunden. Während der Behandlung werden das Blut des Patienten und die Dialysierflüssigkeit an beiden Seiten der semipermeablen Membran im Allgemeinen im Gegenstrom mit einer vorgegebenen Flussrate vorbeigeführt. Die harnpflichtigen Stoffe diffundieren durch die Membran von der Blutkammer in die Kammer für Dialysierflüssigkeit, während gleichzeitig im Blut und in der Dialysierflüssigkeit vorhandene Elektrolyte von der Kammer höherer Konzentration zur Kammer niedrigerer Konzentration diffundieren. Wird an der Dialysemembran ein Druckgradient von der Blutseite zur Dialysatseite aufgebaut, beispielsweise durch eine Pumpe, die flussabwärts des Dialysefilters auf der Dialysatseite Dialysat aus dem Dialysatkreislauf entzieht, tritt Wasser aus dem Patientenblut über die Dialysemembran in den Dialysatkreislauf über. Dieser Vorgang der Ultrafiltration führt zu einer gewünschten Entwässerung des Patientenbluts.

Bei der Hämofiltration wird dem Patientenblut durch Anlegen eines Transmembrandrucks im Dialysator Ultrafiltrat entzogen, ohne dass Dialysierflüssigkeit auf der dem Patientenblut gegenüber liegenden Seite der Membran des Dialysators vorbeigeführt wird. Zusätzlich kann dem Patientenblut eine sterile und pyrogenfreie Substituatslösung zugesetzt werden. Je nachdem, ob diese Substituatslösung stromaufwärts des Dialysators zugesetzt wird oder stromabwärts, spricht man von Prä- oder Postdilution. Der Stoffaustausch erfolgt bei der Hämofiltration konvektiv.

Die Hämodiafiltration kombiniert die Verfahren der Hämodialyse und der Hämofiltration. Es findet sowohl ein diffusiver Stoffaustausch zwischen Patientenblut und Dialysierflüssigkeit über die semipermeable Membran eines Dialysators statt, als auch eine Abfiltrierung von Plasmawasser durch einen Druckgradienten an der Membran des Dialysators.

Die Verfahren der Hämodialyse, der Hämofiltration und der Hämodiafiltration, im Folgenden unter dem Begriff Hämodialyse zusammengefasst, werden in der Regel mit automatischen Hämodialysegeräten durchgeführt, wie sie beispielsweise von der Anmelderin unter der Bezeichnung 5008 vertrieben werden.

Die Plasmapherese ist ein Blutbehandlungsverfahren, bei dem das Patientenblut in das Blutplasma und seine korpuskularen Bestandteile (Zellen) aufgetrennt wird. Das abgetrennte Blutplasma wird gereinigt oder durch eine Substitutionslösung ersetzt und das gereinigte Blutplasma oder die Substitutionslösung dem Patienten zurückgegeben.

Bei der Peritonealdialyse wird die Bauchhöhle eines Patienten über einen durch die Bauchdecke geführten Katheter mit einer Dialyseflüssigkeit befüllt, die ein Konzentrationsgefälle gegenüber den körpereigenen Flüssigkeiten aufweist. Über das als Membran wirkende Bauchfell (Peritoneum) treten die im Körper vorliegenden Giftstoffe in die Bauchhöhle über. Nach einigen Stunden wird die sich in der Bauchhöhle des Patienten befindliche, nunmehr verbrauchte Dialyseflüssigkeit ausgetauscht. Durch osmotische Vorgänge kann Wasser aus dem Blut des Patienten über das Bauchfell in die Dialyseflüssigkeit übertreten und den Patienten somit entwässern.

Das Verfahren zur Peritonealdialyse wird in der Regel mit Hilfe von automatischen Peritonealdialysegeräten, wie sie beispielsweise von der Anmelderin unter der Bezeichnung sleep.safe vertrieben werden, durchgeführt.

Dialysegeräte, als Beispiel für komplexe medizinische Geräte, haben umfangreiche Funktionen. Um diese Funktionen zu steuern, sind medizinische Geräte wie Dialysegeräte mit zumindest einer Steuervorrichtung ausgerüstet. Diese können als CPU (central processing unit) oder Mikrokontroller ausgeführt sein, die von Softwareprogrammen zur Durchführung der Funktionen programmiert werden. Die Bedienung solcher Geräte geschieht häufig über Touchscreendisplays. Ein solches Touchscreendisplay kombiniert in einer gemeinsamen Oberfläche eine Ein- und eine Ausgabevorrichtung, in dem es eine berührungsempfindliche Oberfläche bereitstellt, mit der Bedienereingaben detektierbar sind.

Mögliche Ausführungen sehen die räumliche Trennung von Ein- und Ausgabevorrichtung vor, beispielsweise durch ein konventionelles Display, beispielsweise als CRT-Monitor (Cathode Ray Tube), LCD (Liquid Christal Display), Plasma- oder OLED-Display (Organic Light Emitting Device) ausgeführt, als Ausgabevorrichtung und einem davon räumlich getrennten Touchpad, das eine berührempfindliche Oberfläche bereitstellt, mit der Bedienereingaben detektierbar sind, als Eingabevorrichtung.

Die Behandlung von Patienten in Dialyseeinrichtungen erfordert es, dass die entsprechenden Dialysemaschinen von speziell geschultem Bedienpersonal eingerichtet und eingestellt werden. Da in einer üblichen Dialysestation Dialysemaschinen unterschiedlicher Bauart vorhanden sein können, kann es vorkommen, dass bestimmte Dialyseschwestern nicht auf allen Dialysegeräten geschult sind und entsprechend eine Bedienung, besonders eine Bedienung kritischer Parameter, nur durch entsprechend geschultes Personal durchgeführt werden soll. Bisher wurde dies lediglich dadurch überwacht, dass dem entsprechenden Dialysepersonal eine Bedienung der entsprechenden Dialysegeräte untersagt wurde.

Weiterhin soll vermieden werden, dass die Patienten selbst, oder Besucher oder Dritte die Dialysegeräte während eines Dialysevorganges absichtlich oder unbeabsichtigt manipulieren können. Hier wurde bislang nur auf eine Überwachung der entsprechenden Räumlichkeiten gesetzt.

Aus der EP 1 576 972 A2 ist ein Verfahren bekannt, welches sich generell auf das Identifizieren von Pflegepersonal bezieht, um Zugangsberechtigungen zu überprüfen und gegebenenfalls Einstellungen und Parameter eines medizinischen Geräts für Änderungen freizuschalten.

Weiterhin sind aus der US 2012/075061 A1 Verfahren bekannt, wobei eine Konfiguration eines medizinischen Geräts oder eines Medikaments entsprechend einer Zuordnung eines automatisch identifizierten Patienten und Arztes angepasst wird, um eine Freigabe von Zugangsberechtigungen und Parameter eines medizinischen Geräts zu ermöglichen.

Ebenfalls zeigt die US 2004/172222 A1 ein Verfahren, wobei eine verbesserte Darstellung von Patienteninformationen und, im Falle einer Notsituation eines Patienten, ein Aussenden eines Alarms an medizinisches Personal vorgesehen sind.

### Darstellung der Erfindung

Es ist eine Aufgabe der vorliegenden Erfindung, die Bediensicherheit von medizinischen Geräten zu verbessern und entsprechend die Patientensicherheit zu erhöhen.

Diese Aufgabe wird gelöst durch ein Behandlungssystem für einen Patienten mit den Merkmalen des Anspruchs 1. Vorteilhafte Weiterbildungen ergeben sich aus den Unteransprüchen.

Entsprechend wird ein Behandlungssystem für einen Patienten vorgeschlagen, bevorzugt zur Durchführung einer Dialysebehandlung, umfassend ein medizinisches Gerät, bevorzugt ein Dialysegerät, welches eine Kommunikationsvorrichtung aufweist, welche dazu eingerichtet ist, ein Abfragesignal drahtlos auszusenden und ein Identifikationssignal drahtlos zu empfangen, sowie eine von einem Bediener tragbare Identifikationsvorrichtung zur Identifikation des Bedieners, wobei die Identifikationsvorrichtung dazu eingerichtet ist, ein Identifikationssignal drahtlos an das medizinische Gerät zu senden und wobei die Identifikationsvorrichtung dazu eingerichtet ist, zyklisch und/oder nach Empfang eines Abfragesignals von der Kommunikationsvorrichtung des medizinischen Geräts ein Identifikationssignal auszusenden und Informationen, die von der Kommunikationsvorrichtung gesendet werden, zu empfangen, wobei die Kommunikationsvorrichtung dazu eingerichtet ist, das Identifikationssignal darauf zu prüfen, ob es das Identifikationssignal eines Wartungsmitarbeiters ist, und wenn das Identifikationssignal das Identifikationssignal eines Wartungsmitarbeiters ist, und wenn von dem medizinischen Gerät ein Wartungsbedarf festgestellt wurde, eine dem Wartungsbedarf entsprechende Alarmierung oder eine dem Wartungsbedarf entsprechende Information an die Identifikationsvorrichtung zu senden.

Durch die Bereitstellung der Identifikationsvorrichtung, welche zur drahtlosen Übertragung eines Identifikationssignals an das medizinische Gerät dient, ist es möglich, Identifikationsdaten von einem bestimmten Bediener beziehungsweise von Bedienpersonal an das jeweilige zu bedienende medizinische Gerät zu übertragen. Entsprechend kann im medizinischen Gerät dann aufgrund des empfangenen Signals entweder eine Freigabe von Bedienparametern erfolgen, oder aber eine Änderung von Bedienparametern blockiert werden.

Auf diese Weise wird es möglich, jeden autorisierten Bediener eine entsprechende Identifikationsvorrichtung tragen zu lassen, derart, dass der entsprechende Bediener dann nur die medizinischen Geräte bedienen kann, für welche er auch zertifiziert beziehungsweise autorisiert ist. Auf diese Weise lässt sich vermeiden, dass ein medizinisches Gerät von einem Bediener bedient wird, welcher die entsprechende Qualifikation beziehungsweise Einweisung auf dem jeweiligen Gerätemuster nicht besitzt. Weiterhin kann auf diese Weise auch vermieden werden, dass ein Patient selbst oder aber Besucher oder Dritte, welche auf einer entsprechenden medizinischen Station, insbesondere einer Dialysestation anwesend sind, bewusst oder versehentlich Änderungen an den Parametern des medizinischen Geräts vornehmen.

Weiterhin wird es möglich, die von dem jeweiligen, qualifizierten Bedienern vorgenommenen Änderungen zu protokollieren und entsprechend eine durchgeführte Parameteränderung einem spezifischen Bediener zuzuordnen. Auf diese Weise lässt sich über ein Protokoll, welches beispielsweise in dem jeweiligen medizinischen Gerät geführt wird, erreichen, dass die Behandlung des jeweiligen Patienten lückenlos dokumentiert wird und nicht nur Zeitpunkt und Umfang von Parameteränderungen dokumentiert wird, sondern auch dokumentiert werden kann, durch welchen Bediener die entsprechenden Parameteränderungen durchgeführt wurden.

Die Vorrichtung zur Identifikation eines Bedieners ist bevorzugt so ausgestaltet, dass sie von einem Bediener problemlos mitgeführt werden kann. Dies kann beispielsweise eine ähnlich einer Armbanduhr ausgebildete Form sein, aber auch in Form beispielsweise eines an der Kleidung oder einem Gürtel anhaftbaren, einem Pager ähnlichen Gerät, oder aber auch in Form von entsprechenden Mobiltelefonen, Smartphones, oder tragbaren Computern, welches jeweils individualisiert für einen bestimmten Bediener vorliegen.

Besonders bevorzugt ist die Kommunikationsvorrichtung dazu eingerichtet, ein individuelles Identifikationssignal für jeden einzelnen Bediener zu übertragen. Entsprechend kann eine jede Vorrichtung zur Identifikation eines Bedieners entsprechend für den jeweiligen Bediener individualisiert werden. Hierzu kann es entweder notwendig sein, dass sich der jeweilige Bediener, beispielsweise zu Beginn seiner Schicht, an der Vorrichtung anmeldet, beispielsweise durch die Eingabe einer Bedienerkennung und eines entsprechenden Passwortes beziehungsweise eines entsprechenden PIN-Codes (PIN steht für "Persönliche Identifikationsnummer" oder "personal identification number"). Die Verbindung aus Nutzerkennung und PIN schaltet entsprechend die Identifikationsvorrichtung frei, so dass der entsprechende Bediener, welcher die Vorrichtung bei sich trägt, an entsprechenden medizinischen Geräten, zu deren Bedienung er qualifiziert und zugelassen ist, entsprechende Parameteränderungen vornehmen kann.

Die Vorrichtung ist besonders bevorzugt so ausgebildet, dass die Identifikationsvorrichtung autark mit Energie versorgt wird, beispielsweise über eine entsprechende Batterie oder einen Akkumulator. Damit ist ein besonders flexibler Einsatz der Vorrichtung möglich, so dass der Tragekomfort für den Bediener gewährleistet ist.

In einer Alternative kann die Vorrichtung zur drahtlosen Übertragung eines Identifikationssignals in Form beispielsweise eines passiven RFID-Chips (RFID steht für "radio-frequency identifcation") ausgebildet sein, welcher auf Anforderung des medizinischen Geräts hin eine entsprechende Kennung ausgibt.

In einer besonders bevorzugten Variante umfasst die Vorrichtung weiterhin eine Alarmierungsvorrichtung, welche dazu eingerichtet ist, einen Bediener über einen bestimmten kritischen Zustand eines medizinischen Geräts zu informieren beziehungsweise zu alarmieren. Hierzu ist es vorgesehen, dass das medizinische Gerät seinerseits ein entsprechendes Alarmsignal an die Identifikationsvorrichtung, welche von dem jeweiligen Bediener getragen wird, übersendet und auf diese Weise eine zusätzliche Alarmierung des Bedieners durchführt. Hierzu kann es auch vorgesehen sein, dass an sämtliche, im Umkreis vorliegenden Bediener eine solche Alarmmeldung ausgegeben wird. Besonders bevorzugt wird dabei auf der Vorrichtung zur Identifikation eines Bedieners gleichzeitig der entsprechende Standort des alarmierenden medizinischen Geräts ausgegeben, sowie eine Angabe des jeweiligen Alarmgrundes.

Zur Individualisierung der jeweiligen Identifikationsvorrichtung können neben softwaregestützten Individualisierungsmerkmalen auch andere maschinenlesbare, individuelle Codes, welche auf Anforderung des medizinischen Geräts hin von der Identifikationsvorrichtung zum Identifizieren des Bedieners an das medizinische Gerät übertragen werden, vorgesehen sein, so wie beispielsweise feste IP-Adressen oder MAC-Adressen, welche für die Identifikationsvorrichtung spezifisch sind.

Ein Behandlungssystem umfasst entsprechend ein medizinisches Gerät, bevorzugt ein Dialysegerät, und eine Identifikationsvorrichtung, welche so eingerichtet ist, dass sie mit dem medizinischen Gerät drahtlos kommunizieren kann. Über eine Anfrage des medizinischen Geräts, welche bevorzugt in kurzen Zeitintervallen und beispielsweise alle 0,5 Sekunden stattfindet, wird eine sich in der näheren Umgebung des medizinischen Geräts befindliche Identifikationsvorrichtung dazu angestoßen, ein entsprechendes Identifikationssignal an das medizinische Gerät zu senden.

Die Identifikationsvorrichtung kann dabei beispielsweise in Form eines Pagers, eines Handies oder eines mobilen Computers, insbesondere eines Tablet-PC, ausgebildet sein und ist besonders bevorzugt in Form einer direkt am Körper tragbaren Vorrichtung ausgebildet, so wie beispielsweise in Form einer Armbanduhr, welche gleichzeitig die Identifikationsfunktion übernimmt.

Sowohl in dem medizinischen Gerät als auch in der Identifikationsvorrichtung sind entsprechende Kommunikationsvorrichtungen vorgesehen, welche eine Kommunikation des medizinischen Geräts mit der Identifikationsvorrichtung erlauben. Die Kommunikation findet dabei drahtlos und bevorzugt über elektromagnetische Wellen, wie beispielsweise WLAN, Bluetooth beziehungsweise andere Funkübertragungsprotokolle statt. Eine Kommunikation über eine Infrarotverbindung kann ebenso vorgesehen sein, wobei eine Kommunikation über eine Infrarotverbindung nur bei einer direkten Sichtverbindung zwischen dem medizinischen Gerät und der Identifikationsvorrichtung sicher funktioniert. Entsprechend kann die Bereitstellung einer Infrarotverbindung auch zusätzlich zu einer Funkverbindung vorgesehen sein, um hier einen redundanten Kommunikationskanal bereitzustellen und eine noch genauere Identifikation eines Bedieners zu ermöglichen.

Im Normalbetrieb kommunizieren das medizinische Gerät und die Identifikationsvorrichtung mit einer geringen Sendeleistung miteinander. Im normalen Betrieb ist es auch nur notwendig, den sich unmittelbar in der Nähe des Geräts befindlichen Bediener eindeutig zu identifizieren und entsprechend die jeweiligen Bedienelemente an dem medizinischen Gerät freizugeben oder zu sperren. Insbesondere ist das medizinische Gerät hier so eingerichtet, dass eine Veränderung bestimmter Parameter, insbesondere eine Veränderung kritischer Parameter, nur dann an dem medizinischen Gerät durchgeführt werden können, wenn eine ordnungsgemäße Identifizierung des jeweiligen Bedieners vorliegt. Auf diese Weise kann verhindert werden, dass unqualifizierte oder nicht autorisierte Bediener eine Veränderung der jeweiligen Parameter vornehmen oder eine zufällige beziehungsweise unabsichtliche Veränderung von Parametern stattfindet.

Der Bediener befindet sich, wenn er eine Bedienung des medizinischen Geräts vornehmen möchte, in unmittelbarer Nähe des medizinischen Geräts, um es bedienen zu können.

Das medizinische Gerät kann eine Abfrage nach dem Bediener entweder ständig durchführen, beispielsweise durch das Aussenden eines entsprechenden Abfragecodeworts in einer bestimmten Periode, beispielsweise alle 0,5 Sekunden. Eine Abfrage kann aber auch erst dann angestoßen werden, wenn bestimmte Bedienelemente des medizinischen Geräts bedient werden. Die Bedienung der jeweiligen Bedienelemente kann dabei entweder über die tatsächliche Bedienung der Bedienelemente, beispielsweise Taster, Touchscreens oder Stellknöpfe, ausgelöst werden, oder aber dadurch, dass die entsprechenden Bedienelemente zusätzlich berührungsempfindlich ausgebildet sind. Entsprechend wird bereits bei einer Berührung der jeweiligen Bedienelemente durch den Bediener, beispielsweise über einen kapazitiven Berührungssensor, eine entsprechende Abfrage der Kommunikationsvorrichtung in dem medizinischen Gerät ausgelöst, so dass festgestellt wird, ob die Bedienung tatsächlich von einem identifizierbaren und autorisierten Bediener durchgeführt wird, oder aber versehentlich oder von einem nicht qualifizierten Bediener durchgeführt wird. In einer Variante kann auch über die Steuervorrichtung des medizinischen Geräts bei Vorliegen einer entsprechenden Bedieneranforderung zur Änderung eines Parameters die Abfrage nach der Qualifikation beziehungsweise der Autorisierung eines Bedieners angestoßen werden.

In einer weiteren bevorzugten Variante ist die Kommunikationsvorrichtung, welche in dem medizinischen Gerät vorgesehen ist, so eingerichtet, dass sie, neben der zyklischen Abfrage beziehungsweise der über eine versuchte Bedienaktion ausgelösten Abfrage von sich in der Nähe befindlichen Identifikationsvorrichtungen, weiterhin einen zweiten Betriebsmodus aufweist, in welchem mit einer deutlich höheren Sendeleistung ein Alarmsignal an die umgebenden Identifikationsvorrichtungen ausgegeben wird. Hierzu wird die Sendeleistung der Kommunikationsvorrichtung in dem medizinischen Geräts drastisch erhöht, um möglichst viel medizinisches Personal erreichen zu können, welches sich in der Regel nicht in direkter Nähe zum medizinischen Gerät aufhält. Hier kann, neben der eigentlichen Alarmnachricht, auch der Standort des jeweiligen alarmierenden medizinischen Geräts sowie der Grund für den Alarm übermittelt werden, damit sich das medizinische Personal vorab auf die zu erwartende Situation einstellen kann. Beispielsweise kann eine Dialyseschwester über die entsprechende Alarmierung, beispielsweise über eine schwerwiegende medizinische Störung wie beispielsweise eine Kreislaufentgleisung eines Patienten, informiert werden, so dass diese gleichzeitig auch sofort einen Arzt hinzuziehen kann, anstatt sich zunächst zum Patienten zu begeben, um die Situation zu überprüfen und erst dann einen Arzt zu alarmieren. Auf diese Weise kann auch wertvolle Zeit gespart werden, wenn die Dringlichkeit des jeweiligen Alarms beziehungsweise der jeweilige Alarmgrund mit übermittelt wird.

Die oben beschriebene Aufgabe wird weiterhin durch ein medizinisches Gerät mit den Merkmalen des Anspruchs 6 gelöst. Entsprechend ist das medizinische Gerät bevorzugt ein Dialysegerät und ist für das oben beschriebene Behandlungssystem vorgesehen. Erfindungsgemäß ist eine Kommunikationsvorrichtung dazu eingerichtet, ein Abfragesignal drahtlos auszusenden und ein Identifikationssignal drahtlos von einer von einem Bediener getragenen Identifikationsvorrichtung zu empfangen, wobei die Kommunikationsvorrichtung dazu eingerichtet ist, das Identifikationssignal darauf zu prüfen, ob es das Identifikationssignal eines Wartungsmitarbeiters ist, und wenn das Identifikationssignal das Identifikationssignal eines Wartungsmitarbeiters ist, und wenn von dem medizinischen Gerät ein Wartungsbedarf festgestellt wurde, eine dem Wartungsbedarf entsprechende Alarmierung oder eine dem Wartungsbedarf entsprechende Information an die Identifikationsvorrichtung zu senden. Auf diese Weise lassen sich die oben beschriebenen Vorteile bezüglich einer Identifizierung eines Bedieners erreichen.

Neben einer tragbaren Identifikationsvorrichtung zur Identifikation eines Bedieners eines medizinischen Geräts, wie in dem oben beschriebenen Behandlungssystem, kann eine Kommunikationsvorrichtung vorgesehen sein, welche dazu eingerichtet ist, ein Abfragesignal drahtlos zu empfangen und ein Identifikationssignal drahtlos an ein medizinisches Gerät in Antwort auf das Abfragesignal auszusenden. Auch auf diese Weise lassen sich die oben beschriebenen Vorteile einer Identifikation eines Bedieners erreichen.

Weiterhin kann die Kommunikationsvorrichtung dazu eingerichtet sein, zyklisch ein Identifikationssignal drahtlos auszusenden, bevorzugt mit einer niedrigen Sendeleistung. Auf diese Weise lässt sich eine Identifizierung eines Bedieners durch ein medizinisches Gerät erzwingen, beispielsweise zur Identifizierung von Wartungspersonal.

Mittels eines Verfahrens mit den Merkmalen des Anspruchs 7 kann ein Bediener eines medizinischen Geräts des oben beschriebenen Behandlungssystems identifiziert werden. Entsprechend sind die Schritte des drahtlosen Aussendens eines Abfragesignals durch eine Kommunikationsvorrichtung des medizinischen Geräts an mindestens eine von einem Wartungsmitarbeiter getragenen und diesen identifizierende Identifikationsvorrichtung, des Empfangens eines in Antwort auf das empfangene Abfragesignal drahtlos ausgesendeten Identifikationssignals in der Kommunikationsvorrichtung des medizinischen Geräts, sowie des Identifizierens des Bedieners auf Grundlage des empfangenen Identifikationssignals, wobei die Kommunikationsvorrichtung dazu eingerichtet ist, einen Alarm oder eine Information an den Wartungsmitarbeiter zu senden, wenn von dem medizinischen Gerät ein Wartungsbedarf festgestellt wurde, vorgesehen.

Weiterhin können die Schritte des Empfangens eines von einem medizinischen Gerät drahtlos ausgesendeten Abfragesignals in einer Kommunikationsvorrichtung der Identifikationsvorrichtung, sowie des drahtlosen Aussendens eines Identifikationssignals in Antwort auf das empfangene Abfragesignal durch die Kommunikationsvorrichtung der Identifikationsvorrichtung vorgesehen.

Ebenfalls kann vorgesehen sein, dass das Aussenden eines Identifikationssignals von einer von einem Bediener getragenen und diesen identifizierenden Identifikationsvorrichtung ein zyklisches Aussenden eines drahtlosen Identifikationssignals zum Empfang in der Kommunikationsvorrichtung eines medizinischen Geräts umfasst. Auch auf diese Weise lässt sich eine Identifizierung eines Bedieners durch ein medizinisches Gerät erzwingen, beispielsweise zur Identifizierung von Wartungspersonal

Mittels eines Computerprogrammprodukts kann das genannte Verfahren in einem medizinischen Gerät eines oben beschriebenen Behandlungssystems ausgeführt werden. Das Computerprogrammprodukt wird dabei bevorzugt in einer Steuervorrichtung des medizinischen Geräts ausgeführt, um die Identifikation eines Bedieners zu erreichen.

Mittels eines Computerprogrammprodukts kann das oben genannte Verfahren auch in einer Identifikationsvorrichtung eines oben beschriebenen Behandlungssystems ausgeführt werden. Dieses Computerprogrammprodukt eignet sich besonders auch zur Ausführung des Verfahrens zum Aussenden eines Identifikationssignals auf einem bestehenden Mobiltelefon, Smartphone und/oder tragbaren Computer, insbesondere einem Tablet-PC.

Generell verfügen das medizinische Gerät und ein Computer im Einklang mit der Lehre der vorliegenden Erfindung über spezifische Programmierungen, die die Bedienung des medizinischen Geräts oder die Anzeige von Daten des medizinischen Geräts oder einer damit ausgeführten Behandlung und/oder die Verwendung von Sensoren zur Überwachung und Steuerung des medizinischen Geräts oder einer damit ausgeführten Behandlung betreffend entsprechend steuern und die beschriebenen Verfahren ausführen.

Diese spezifischen Programmierungen sind Computerprogramme und können direkt in einen internen Speicher des Computers und/oder des medizinischen Geräts geladen werden und Softwarecodeabschnitte umfassen, die die beschriebenen Verfahren ausführen, wenn die Programme auf dem Computer oder dem medizinischen Gerät ablaufen. Die Computerprogramme können als Computerprogrammprodukte, die computerlesbare Programmmittel umfassen, auf Datenmedien vorgehalten werden. Diese Datenmedien sind in einem Computer einsetzbar und umfassen neben physikalischen Speichern, wie Disketten, CD-Roms, Speicherkarten, USB Sticks oder DVDs auch die Speicherung innerhalb von Netzwerken, wie dem Internet, auf die der Bediener Zugriff haben kann.

### Kurze Beschreibung der Figuren

Bevorzugte weitere Ausführungsformen und Aspekte der vorliegenden Erfindung werden durch die nachfolgende Beschreibung der Figuren näher erläutert. Dabei zeigen:
- Figur 1: ein herkömmliches medizinisches Gerät in Form eines Hämodialysegeräts;
- Figur 2: eine schematische Darstellung eines Behandlungssystems mit einem medizinischen Gerät und einer Identifikationsvorrichtung; und
- Figur 3: eine schematische Darstellung des medizinischen Geräts und einer Identifikationsvorrichtung in zwei unterschiedlichen Abständen.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente mit identischen Bezugszeichen bezeichnet und auf eine wiederholte Beschreibung dieser Elemente wird in der nachfolgenden Beschreibung teilweise verzichtet, um Redundanzen zu vermeiden.

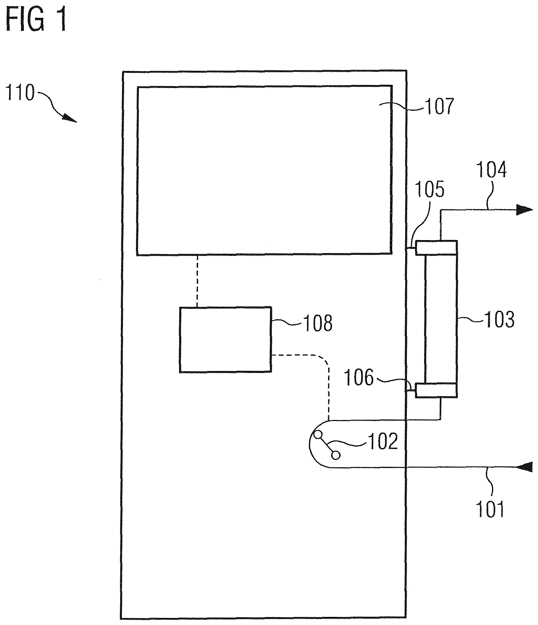

In Figur 1 zeigt schematisch eine Ausführungsform eines herkömmlichen medizinischen Gerätes 110 als Hämodialysegerät bzw. einer Dialysemaschine mit einem Touchscreendisplay 107. Das medizinische Gerät 110 weist umfangreiche Funktionen auf, zu deren Steuerung eine Steuervorrichtung 108 vorgesehen ist. Diese Steuervorrichtung 108 kann als CPU (central processing unit) oder Mikrokontroller ausgeführt sein, die, beispielsweise mittels auf dieser ablaufender Softwareprogramme, dazu eingerichtet ist, die Funktionen zu steuern oder auszuführen. In der Figur 1 ist schematisch eine Anbindung der Steuervorrichtung 108 an eine Blutpumpe 102 gezeigt, wodurch die Steuervorrichtung 108 entsprechend die Blutpumpe 102 ansteuern kann. Das Touchscreendisplay 107 dient unter anderem der Ansteuerung der Steuervorrichtung 108 derart, dass ein Bediener bestimmte Anweisungen, Parameter oder Ablaufprogramme der Steuervorrichtung 108 eingeben und/oder auswählen kann. Das medizinische Gerät 110 zeigt andeutungsweise Teile eines extrakorporalen Blutkreislaufs mit einer arteriellen Blutleitung 101, die Blut eines Patienten (nicht dargestellt) ableitet. Die Blutpumpe 102 fördert das Blut durch einen Dialysefilter 103, der mit einer semipermeablen Membran ausgestattet ist, die den extrakorporalen Blutkreislauf von einem Dialysatkreislauf semipermeabel trennt. Über die venöse Leitung 104 wird das behandelte Blut dem Patienten zurückgegeben. Über die Dialysatleitungen 105 und 106 wird Dialysat durch den Dialysefilter 103 gepumpt, wo es über die semipermeable Membran des Dialysefilters 103 zu einem diffusiven Stoffaustausch mit dem Blut des Patienten kommt. Wird zusätzlich ein Druckgradient von der Blutseite des Dialysefilters zur Dialysatseite des Patienten aufgebaut, wird Plasmawasser aus dem Blut in das Dialysat abgepresst. Das abgepresste Plasmawasser wird auch Ultrafiltrat genannt. Das Blut des Patienten kann so entwässert werden. Das Dialysat wird in dem Hämodialysegerät 110 hergestellt und nach Gebrauch verworfen.

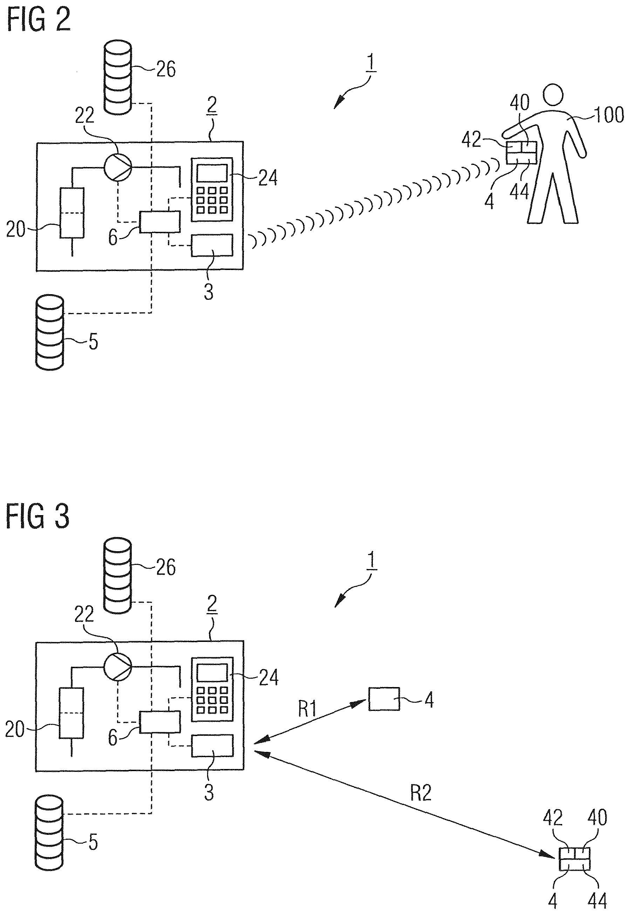

Figur 2 zeigt schematisch ein Behandlungssystem 1, welches ein medizinisches Gerät 2 in Form einer schematisch angedeuteten Dialysevorrichtung mit einem Filter 20, einer Schlauchpumpe 22 und einer Bedienvorrichtung 24 umfasst. Eine Steuervorrichtung 6 ist vorgesehen, welche zur Steuerung der unterschiedlichen Funktionen des medizinischen Geräts 2 eingerichtet ist. Die Steuervorrichtung 6 ist dabei bevorzugt in Form einer CPU ausgebildet, welche über entsprechende Softwareprogramme zur Steuerung und Ausführung der unterschiedlichen Funktionen eingerichtet ist.

Weiterhin ist in dem medizinischen Gerät 2 eine Kommunikationsvorrichtung 3 vorgesehen, welches sowohl Signale aussenden kann, als auch Signale empfangen kann. Die Kommunikationsvorrichtung 3 ist an die Steuervorrichtung 6 angebunden und kann von dieser angesteuert werden. Die Steuervorrichtung 6 und/oder die Kommunikationsvorrichtung 3 sind dazu konfiguriert, ein drahtloses Abfragesignal auszusenden.

Das Behandlungssystem 1 umfasst weiterhin eine Identifikationsvorrichtung 4, welche in dem in Figur 1 schematisch gezeigten Beispiel am Gürtel eines Bedieners 100 getragen wird und entsprechend vom Bediener 100 problemlos mitgeführt werden kann.

Der Bediener 100 des medizinischen Geräts 2 ist üblicherweise eine Dialyseschwester oder eine andere Pflegekraft, welche auf dem jeweiligen medizinischen Gerät 2 eingewiesen ist und entsprechend zur Bedienung des medizinischen Geräts 2 zugelassen ist.

Da in einer klinischen Einrichtung oder einer Dialysestation üblicherweise unterschiedliche Gerätetypen medizinischer Geräte 2 vorgesehen sind, kann es notwendig sein, nur solchen Bedienern 100 den Zugang beziehungsweise die Manipulation bestimmter Parameter oder der gesamten Bedienung eines medizinischen Geräts 2 zu erlauben, welche für speziell diesen Gerätetypus trainiert sind.

Weiterhin erhöht es die Behandlungssicherheit für die behandelten Patienten, wenn nur qualifizierte Bediener 100 die Bedienung des medizinischen Geräts 2 durchführen dürfen und eine Bedienung durch den Patienten selbst oder durch möglicherweise in der klinischen Einrichtung oder der Dialysestation anwesende Besucher oder Dritte ausgeschlossen wird. Dadurch wird auch eine unbeabsichtigte Bedienung durch Besucher oder Dritte, die etwa unvorsichtig an das medizinische Gerät 2 stoßen, vermieden.

Entsprechend ist in dem medizinischen Gerät 2 die Kommunikationsvorrichtung 3 vorgesehen, welche - beispielsweise in Kombination mit der Steuervorrichtung 6 - dazu konfiguriert ist, zyklisch ein drahtloses Abfragesignal, beispielsweise in Form eines Codeworts, auszusenden, wobei die Kommunikationsvorrichtung 3 dann mit einer geringen Sendeleistung betrieben wird, beispielsweise einer Sendeleistung im Milliwattbereich. Wenn sich der direkten Umgebung des medizinischen Geräts 2 ein Bediener 100 befindet, welcher eine entsprechende Identifikationsvorrichtung 4 trägt, kann eine in der Identifikationsvorrichtung 4 vorgesehene Kommunikationsvorrichtung 40 dieses drahtlose Abfragesignal empfangen. Die Identifikationsvorrichtung 4 ist entsprechend dazu konfiguriert, das drahtlose Abfragesignal zu empfangen. Entsprechend sind die Kommunikationsvorrichtung 3 und die Identifikationsvorrichtung 4 dazu eingerichtet, mit den gleichen Signaltypen zu kommunizieren, also beispielsweise im gleichen Frequenzband unter Verwendung des gleichen Kommunikationsprotokolls. Als Antwort auf das Abfragesignal ist die Kommunikationsvorrichtung 40 dazu eingerichtet, der Identifikationsvorrichtung 4 ein entsprechendes drahtloses Identifikationssignal zu senden, beispielsweise ein Codewort. Dieses Codewort wird von der Kommunikationsvorrichtung 3 in dem medizinischen Gerät 2 empfangen und entsprechend ausgewertet.

Für jede in der klinischen Einrichtung oder einer Dialysestation verwendete Identifikationsvorrichtung 4 wird beispielsweise ein individuelles Codewort vergeben. Besonders bevorzugt wird für jeden individuellen Bediener 100 ein eigenes Codewort vergeben. Auf diese Weise kann entweder über eine Zuordnung der Identifikationsvorrichtung 4 zu dem jeweiligen Bediener 100 in einer bestimmten Arbeitsschicht, oder aber durch die eineindeutige Zuordnung des Codeworts zu dem Bediener 100 eine Identifikation des Bedieners vorgenommen werden.

Durch die geringe Sendeleistung der Kommunikationsvorrichtung 3 beim Aussenden des Abfragesignals werden Identifikationsvorrichtungen 4 nur in einem geringen Umkreis um die Kommunikationsvorrichtung 3 herum angesprochen, beispielsweise in dem in Figur 2 gezeigten Abstand R1 von ungefähr 1m bis 5m, bevorzugt einem Meter.

Befindet sich entsprechend ein Bediener 100 in der unmittelbaren Nähe, beispielsweise innerhalb eines Abstandes R1 von 1m - 5m um das medizinische Gerät 2 herum, so kann das medizinische Gerät 2 aus dem von der Identifikationsvorrichtung 4 ausgesendeten und von der Kommunikationsvorrichtung 3 empfangenen Identifikationssignal erkennen, um welchen konkreten Bediener es sich handelt.

Die Steuerung des medizinischen Geräts 3 zur Durchführung der genannten Identifizierung eines Bedieners 100 wird bevorzugt durch ein in der Steuervorrichtung 6 des medizinischen Geräts 3 ablaufendes Computerprogrammprodukt erreicht.

Bevorzugt kann das medizinische Gerät 2 einen entsprechenden Abgleich mit einer Berechtigungsdatenbank 5 vornehmen, welche entweder direkt mit der Kommunikationsvorrichtung 3 oder mit der Steuervorrichtung 6 verbunden ist und entsprechend in dem medizinischen Gerät 2 vorliegt, oder aber über ein externes Netzwerk zugreifbar ist. Das medizinische Gerät 2 stellt entsprechend auf Grundlage des von der Identifikationsvorrichtung 4 empfangenen Identifikationssignals eine Anfrage an die Berechtigungsdatenbank 5 und kann entsprechend feststellen, ob der Bediener 100, dem das entsprechende Identifikationssignal zugeordnet ist, für die Bedienung des spezifischen medizinischen Geräts 2 zugelassen ist, oder nicht. Ist der Bediener 100 zugelassen, so kann er sämtliche für ihn vorgesehenen Bedienungsschritte beziehungsweise Parameteränderungen beispielsweise über die Bedieneinheit 24 an dem medizinischen Gerät 2 vornehmen.

Stellt das medizinische Gerät 2 bei der Abfrage der Bedienerberechtigungen aus der Berechtigungsatenbank 5 fest, dass der Bediener 100 zur Bedienung des medizinischen Geräts 2 nicht zugelassen ist, so kann der entsprechende Bediener entweder sämtliche Parameter oder aber ausgewählte Parameter des medizinischen Geräts 2 nicht ändern. Entsprechend werden Bedienoptionen für den nicht autorisierten Bediener gesperrt.

In einer weiteren bevorzugten Variante werden in der Berechtigungsdatenbank 5 unterschiedliche Berechtigungsstufen vergeben. Beispielsweise kann ein Arzt, dessen Identifikationsvorrichtung 4 ein bestimmtes Identifikationssignal an die Kommunikationsvorrichtung 3 übersendet, über die Berechtigungsdatenbank 5 einen vollständigen Zugriff auf das medizinische Gerät 2 erhalten. Eine voll ausgebildete und entsprechend zertifizierte Dialyseschwester kann ebenfalls einen Vollzugriff auf das medizinische Gerät 2 erhalten. Hilfspersonal kann jedoch nur einen eingeschränkten Zugriff, beispielsweise auf ausgewählte Parameter des medizinischen Geräts 2, erhalten. Das medizinische Gerät 2 kann dabei so ausgebildet sein, dass eine Bedienung des medizinischen Geräts 2 über die Bedienvorrichtung 24 ohne das Vorliegen eines gültigen Identifikationssignals überhaupt nicht möglich ist. In einer Variante sind einige Parameter allgemein zugänglich, für die Behandlung und/oder die Patientensicherheit kritische Parameter aber nur identifizierten und autorisierten Bedienern vorbehalten.

In einer Protokolldatenbank 26 werden die durchgeführten Parameteränderungen beziehungsweise sämtliche durchgeführten Bedienschritte des medizinischen Geräts 2 protokolliert. In diesem Zusammenhang wird neben den eigentlichen Bedienschritten und dem Durchführungszeitpunkt dieser Bedienschritte auch protokolliert, welcher Bediener 100 beziehungsweise welche Bediener in der unmittelbaren Umgebung des medizinischen Geräts 2 identifiziert wurden, um so eine direkte Zuordnung von Bedienschritten zu den jeweiligen Bedienern zu erreichen. Auf diese Weise kann die Protokollierung beziehungsweise Dokumentation einer Behandlung noch lückenloser geführt werden.

In einer bevorzugten und besonders effizienten Arbeitsweise trägt der Bediener 100 die Identifikationsvorrichtung 4 beispielsweise in Form einer Armbanduhr. Befindet sich der Bediener 100 in der Nähe des medizinischen Geräts 2, so empfängt die Kommunikationsvorrichtung 40 in der Identifikationsvorrichtung 4 das von der Kommunikationsvorrichtung 3 des medizinischen Geräts 2 zyklisch ausgesendete Signal, wodurch der Mikroprozessor in der Identifikationsvorrichtung 4 aus einem Sleepmodus aufgeweckt wird und seinerseits das Codewort zurücksendet, wobei hier ebenfalls eine sehr geringe Sendeleistung verwendet wird. Nach dem Aussenden des entsprechenden Codewortes wird der Mikroprozessor wieder in den Sleepmodus versetzt. Auf diese Weise kann ein sehr energieeffizienter Betrieb der Identifikationsvorrichtung 4 erreicht werden, so dass eine autarke Energieversorgung der Identifikationsvorrichtung 4, beispielsweise durch einen Akkumulator oder eine Batterie 44, entweder eine besonders lange Laufzeit aufweist, oder so klein dimensioniert werden kann, dass ein hoher Tragekomfort der Identifikationsvorrichtung 4 erreicht werden kann.

Die Identifikationsvorrichtung 4 kann auch durch ein auf einem Mobiltelefon, Smartphone und/oder tragbaren Computer, insbesondere einem Tablet-PC, ablaufendes Computerprogrammprodukt dargestellt werden, wobei das Computerprogrammprodukt entsprechend so auf die Komponenten der genannten Geräte wirkt, dass das Gerät dazu eingerichtet ist, den Empfang des Abfragesignals von der Kommunikationsvorrichtung 3 des medizinischen Geräts 3 zu ermöglichen und auch eine Aussendung des Identifikationssignals in Antwort auf das Abfragesignal zu ermöglichen.

In einer besonders bevorzugten Variante, welche auch in Figur 3 gezeigt ist, wird die Sendeleistung der Kommunikationsvorrichtung 3 des medizinischen Geräts 2 im Falle des Auftretens eines Alarms gegenüber dem Normalbetrieb drastisch angehoben. Hier können beispielsweise Sendeleistungen im Milliwattbereich, beispielsweise 2 Milliwatt, verwendet werden, um Identifikationsvorrichtungen 4, welche sich in einem weiteren Umkreis befinden, beispielsweise dem Umkreis R2 von beispielsweise 20 m um die medizinische Vorrichtung 2 herum, mit einem Alarmsignal zu beaufschlagen. Die Identifikationsvorrichtung 4 weist dabei besonders bevorzugt neben dem Kommunikationsvorrichtung 40 auch eine Alarmierungsvorrichtung 42 auf, welche beispielsweise einen akustischen Alarm und/oder einen optischen Alarm ausgeben kann. In einer besonders bevorzugten Variante wird, neben der eigentlichen Alarmierung, auch der Standort des alarmierenden medizinischen Geräts 2 sowie der Grund der Alarmierung, also beispielsweise der entsprechende alarmierende Parameter, mit übertragen. Entsprechend kann der jeweilige Bediener 100, der die Alarmierung empfängt, sofort anhand des übertragenen Alarmgrundes erkennen, welche medizinische Situation ihn bei dem alarmierenden medizinischen Gerät 2 erwarten wird.

Diese Alarmierung wird üblicherweise durch einen aufgrund der gängigen Sicherheitsnormen bereits vorhandenen Hausruf beziehungsweise Schwesternruf ausgelöst, der zur Alarmierung des medizinischen Personals bei Auftreten eines kritischen Zustands des medizinischen Geräts dient. Die hier vorgestellte Alarmierung über ein an die Identifikationsvorrichtung 4 gesendetes Alarmsignal erhöht die Redundanz der Systeme und ermöglicht gleichzeitig die Übertragung von Zusatzinformationen an das medizinische Personal.

Die drahtlose Kommunikation zwischen der Kommunikationsvorrichtung 3 in dem medizinischen Gerät 2 und der Kommunikationsvorrichtung 40 in der Identifikationsvorrichtung 4 kann über bekannte Kommunikationsstandards und bekannte Kommunikationslösungen durchgeführt werden. Beispielsweise kann über Funkprotokolle wie beispielsweise den Zigbee-Standard ein zuverlässiges und kostengünstiges System aufgebaut werden. Andere Funkprotokolle sind selbstverständlich ebenfalls denkbar, wie WLAN, Bluetooth oder andere, herkömmliche Funkprotokolle.

In einer alternativen Ausbildung kann die Identifikationsvorrichtung 4 auch passiv ausgeführt sein und eine Bedienererkennung über einen RFID Chip ermöglichen. Eine solche Variante führt dazu, dass die Identifkationsvorrichtung 4 ohne Energiezufuhr betrieben werden kann. Ein RFID Chip kann beispielsweise auch in einem Namensschild eines Bedieners aufgenommen sein.

Weiterhin kann eine Infrarotverbindung zwischen der Identifikationsvorrichtung 4 und der Kommunikationsvorrichtung 3 des medizinischen Geräts 2 hergestellt werden, welche dann bevorzugt zusätzlich zu der drahtlosen Funkverbindung einer sicheren Identifikation des jeweiligen Bedieners 100 bei der Bedienung der Bedieneinheit 24 dient. Die Ausbildung einer Infrarotverbindung kann auch als zusätzliche Sicherheit dazu dienen, bei der Identifikation von mehreren Bedienern im Umkreis des medizinischen Geräts 2 denjenigen Bediener zu identifizieren, der tatsächlich Einstellungen an dem medizinischen Gerät 2 vornimmt. Dabei wird hier ausgenutzt, dass die Infrarotverbindung nur bei einer Sichtverbindung aufgebaut werden kann. Wenn sich also andere identifizierte Bediener in der Umgebung des medizinischen Geräts 2 aufhalten, welche beispielsweise gerade ein benachbartes medizinisches Gerät bedienen, aber dennoch auch von dem betrachteten medizinischen Gerät identifiziert werden, sind diese Bediener vom betrachteten medizinischen Gerät abgewendet und können entsprechend keine Infrarotverbindung aufbauen. Damit kann eine noch genauere Zuordnung der Bediener erfolgen.

Wie bereits beschrieben, kann das Aussenden des Signals über die Kommunikationsvorrichtung 3 von dem medizinischen Gerät 2 aus, um das Vorliegen von Identifikationsvorrichtungen 4 im näheren Umkreis abzufragen, entweder zyklisch durchgeführt werden, oder aber es kann immer dann angestoßen werden, wenn eine tatsächliche Bedienung über die Bedieneinheit 24 durchgeführt werden soll. Auf letztere Weise kann die Belastung der Umgebung mit potentiell störenden Signalen weiter reduziert werden.

In einer bevorzugten Variante kann auch die Identifikationsvorrichtung 4 dazu eingerichtet sein, zyklisch ein Identifikationssignal drahtlos auszusenden. Dies wird bevorzugt bei einer niedrigen Sendeleistung durchgeführt. Auf diese Weise wird es möglich, den vorliegenden medizinischen Geräten 2 die Anwesenheit einer bestimmten Identifikationsvorrichtung 4, und damit eines bestimmten Bedieners, mitzuteilen. Beispielsweise kann durch eine solche Mitteilung dann das medizinische Gerät 2 dazu veranlasst werden, eine bestimmte Information an die Identifikationsvorrichtung 4 zu übertragen. In einem Beispiel kann von der Identifikationsvorrichtung 4 eines Wartungsmitarbeiters ein Identifikationssignal zyklisch ausgegeben werden, welches dann von einem medizinischen Gerät 2 empfangen wird. Wurde vorher bei dem medizinischen Gerät 2, beispielsweise über dessen Steuervorrichtung 6, ein Wartungsbedarf festgestellt, kann das medizinische Gerät 2 über seine Kommunikationsvorrichtung 3 eine entsprechende Alarmierung beziehungsweise Information an die Identifikationsvorrichtung 4 des Wartungsmitarbeiters senden. Entsprechend kann beispielsweise eine Wartung direkt durchgeführt werden oder der notwendige Wartungsbedarf kann direkt von dem Wartungsmitarbeiter festgestellt werden.

Soweit anwendbar können alle einzelnen Merkmale, die in den einzelnen Ausführungsbeispielen dargestellt sind, miteinander kombiniert und / oder ausgetauscht werden, ohne den Bereich der Erfindung zu verlassen.

### Bezugszeichenliste

- 1: Behandlungssystem
- 100: Bediener
- 101: arterielle Blutleitung
- 102: Blutpumpe
- 103: Dialysefilter
- 104: venöse Leitung
- 105: Dialysatleitung
- 106: Dialysatleitung
- 107: Touchscreen
- 108: Steuerungsvorrichtung
- 110: medizinisches Gerät nach dem Stand der Technik
- 2: medizinisches Gerät
- 20: Filter
- 22: Schlauchpumpe
- 24: Bedieneinheit
- 26: Protokolldatenbank
- 3: Kommunikationsvorrichtung
- 4: Identifikationsvorrichtung
- 40: Kommunikationsvorrichtung
- 42: Alarmierungsvorrichtung
- 44: Batterie
- 5: Berechtigungsdatenbank
- 6: Steuerungsvorrichtung

- R1: geringer Abstand
- R2: großer Abstand

## Patentansprüche

1. Behandlungssystem (1) für einen Patienten, umfassend:
- ein medizinisches Gerät (2), welches eine Kommunikationsvorrichtung (3) aufweist, welche dazu eingerichtet ist, ein Abfragesignal drahtlos auszusenden und ein Identifikationssignal drahtlos zu empfangen, und
- eine von einem Bediener (100) tragbare Identifikationsvorrichtung (4) zur Identifikation des Bedieners (100), wobei die Identifikationsvorrichtung (4) dazu konfiguriert ist, ein Identifikationssignal drahtlos an das medizinische Gerät (2) zu senden und wobei die Identifikationsvorrichtung (4) dazu eingerichtet ist, zyklisch und/oder nach Empfang eines Abfragesignals von der Kommunikationsvorrichtung (3) des medizinischen Geräts (2) ein Identifikationssignal auszusenden und Informationen, die von der Kommunikationsvorrichtung (3) gesendet werden, zu empfangen,
**dadurch gekennzeichnet, dass**
die Kommunikationsvorrichtung (3) dazu eingerichtet ist, das Identifikationssignal darauf zu prüfen, ob es das Identifikationssignal eines Wartungsmitarbeiters ist, und wenn das Identifikationssignal das Identifikationssignal eines Wartungsmitarbeiters ist, und wenn von dem medizinischen Gerät (2) ein Wartungsbedarf festgestellt wurde, eine dem Wartungsbedarf entsprechende Alarmierung oder eine dem Wartungsbedarf entsprechende Information an die Identifikationsvorrichtung (4) zu senden.

2. Behandlungssystem (1) gemäß Anspruch 1, **dadurch gekennzeichnet, dass** die Identifikationsvorrichtung (4) in Form einer Uhr, eines Pagers, eines Mobiltelefons, oder eines tragbaren Computers ausgebildet ist.

3. Behandlungssystem (1) gemäß einem der vorstehenden Ansprüche, **dadurch gekennzeichnet, dass** die Kommunikationsvorrichtung (3) des medizinischen Geräts (2) und die Identifikationsvorrichtung (4) dazu eingerichtet sind, über einen gemeinsamen Funkstandard miteinander zu kommunizieren und/oder dass die Kommunikationsvorrichtung (3) des medizinischen Geräts (2) und die Identifikationsvorrichtung (4) dazu eingerichtet sind, über ein Infrarotsignal miteinander zu kommunizieren.

4. Behandlungssystem (1) gemäß einem der vorstehenden Ansprüche, **dadurch gekennzeichnet, dass** eine Protokollierungsdatenbank (26) vorgesehen ist und das medizinische Gerät (2) dazu eingerichtet ist, die Bedienzugriffe und die Identitäten der die Bedienzugriffe durchführenden Bediener in der Protokollierungsdatenbank (26) zu protokollieren.

5. Behandlungssystem (1) gemäß einem der vorstehenden Ansprüche, **dadurch gekennzeichnet, dass** das medizinische Gerät (2) ein Dialysegerät ist, welches zur Durchführung einer Dialysebehandlung eingerichtet ist.

6. Medizinisches Gerät (2) für ein Behandlungssystem (1), welches eine Kommunikationsvorrichtung (3) aufweist, welche dazu eingerichtet ist, ein Abfragesignal drahtlos auszusenden und ein Identifikationssignal drahtlos von einer von einem Bediener (100) getragenen Identifikationsvorrichtung (4) zu empfangen,
**dadurch gekennzeichnet, dass**
die Kommunikationsvorrichtung (3) dazu eingerichtet ist, das Identifikationssignal darauf zu prüfen, ob es das Identifikationssignal eines Wartungsmitarbeiters ist, und wenn das Identifikationssignal das Identifikationssignal eines Wartungsmitarbeiters ist, und wenn von dem medizinischen Gerät (2) ein Wartungsbedarf festgestellt wurde, eine dem Wartungsbedarf entsprechende Alarmierung oder eine dem Wartungsbedarf entsprechende Information an die Identifikationsvorrichtung (4) zu senden.

7. Verfahren zur Identifizierung eines Wartungsmitarbeiters (100) eines medizinischen Geräts (2) für ein Behandlungssystem (1) gemäß einem der Ansprüche 1 bis 4, umfassend die Schritte:
• Drahtloses Aussenden eines Abfragesignals durch eine Kommunikationsvorrichtung (3) des medizinischen Geräts (2) an mindestens eine von einem Wartungsmitarbeiter (100) getragenen und diesen identifizierende Identifikationsvorrichtung (4);
• Empfangen eines in Antwort auf das empfangene Abfragesignal drahtlos ausgesendeten Identifikationssignals in der Kommunikationsvorrichtung (3) des medizinischen Geräts (2) ;
• Identifizieren des Wartungsmitarbeiters (100) auf Grundlage des empfangenen Identifikationssignals,
• wobei die Kommunikationsvorrichtung (3) dazu eingerichtet ist, einen Alarm oder eine Information an den Wartungsmitarbeiter (100) zu senden, wenn von dem medizinischen Gerät (2) ein Wartungsbedarf festgestellt wurde.

8. Verfahren gemäß Anspruch 7, **dadurch gekennzeichnet, dass** das medizinische Gerät (2) ein Dialysegerät ist, welches zur Durchführung einer Dialysebehandlung eingerichtet ist.

## Claims

1. Treatment system (1) for a patient, comprising:
- a medical device (2) which comprises a communication device (3) configured to wirelessly send an inquiry signal and to wirelessly receive an identification signal, and
- an identification device (4) that can be carried by an operator (100) to identify the operator (100) wherein the identification device (4) is configured to wirelessly send an identification signal to the medical device (2) and wherein the identification device (4) is configured to send an identification signal cyclically and/or after receiving an inquiry signal from the communication device (3) of the medical device (2) and to receive information sent by the communication device (3),
**characterised in that**
the communication device (3) is configured to check the identification signal to determine whether it is the identification signal of a maintenance employee and if the identification signal is the identification signal of a maintenance employee and if a maintenance requirement has been determined by the medical device (2), to send an alarm corresponding to the maintenance requirement or information corresponding to the maintenance requirement to the identification device (4).

2. Treatment system (1) according to claim 1, **characterised in that** the identification device (4) is designed in the form of a watch, a pager, a mobile telephone or a portable computer.

3. Treatment system (1) according to any one of the preceding claims, **characterised in that** the communication device (3) of the medical device (2) and the identification device (4) are configured to communicate with each other via a common radio standard and/or **in that** the communication device (3) of the medical device (2) and the identification device (4) are configured to communicate with each other via an infrared signal.

4. Treatment system (1) according to any one of the preceding claims, **characterised in that** a recording database (26) is provided and the medical device (2) is configured to record the operating access and the identities of the operators implementing the operating access in the recording database (26).

5. Treatment system (1) according to any one of the preceding claims, **characterised in that** the medical device (2) is a dialysis device which is configured to perform a dialysis treatment.

6. Medical device (2) for a treatment system (1) which comprises a communication device (3) configured to wirelessly send an inquiry signal and to wirelessly receive an identification signal from an identification device (4) carried by an operator (100),
**characterised in that**
the communication device (3) is configured to check the identification signal to determine whether it is the identification signal of a maintenance employee and if the identification signal is the identification signal of a maintenance employee and if a maintenance requirement has been determined by the medical device (2), to send an alarm corresponding to the maintenance requirement or information corresponding to the maintenance requirement to the identification device (4).

7. Method for identifying a maintenance employee (100) of a medical device (2) for a treatment system (1) according to any one of claims 1 to 4, comprising the steps:
• wirelessly sending an inquiry signal by means of a communication device (3) of the medical device (2) to at least one identification device (4) carried by a maintenance employee (100) and identifying said employee;
• receiving an identification signal sent wirelessly in response to the received inquiry signal in the communication device (3) of the medical device (2);
• identifying the maintenance employee (100) based on the identification signal received,
• wherein the communication device (3) is configured to send an alarm or information to the maintenance employee (100) if a maintenance requirement has been determined by the medical device (2).

8. Method according to claim 7, **characterised in that** the medical device (2) is a dialysis device which is configured to perform a dialysis treatment.

## Revendications

1. Système de traitement (1) pour un patient, comprenant :
- un appareil médical (2), lequel présente un dispositif de communication (3), lequel est aménagé pour émettre sans fil un signal d'interrogation et recevoir sans fil un signal d'identification, et
- un dispositif d'identification (4) portatif par un utilisateur (100) pour l'identification de l'utilisateur (100), dans lequel le dispositif d'identification (4) est configuré pour émettre sans fil un signal d'identification à l'appareil médical (2) et dans lequel le dispositif d'identification (4) est aménagé pour émettre un signal d'identification cycliquement et/ou après réception d'un signal d'interrogation du dispositif de communication (3) de l'appareil médical (2) et recevoir des informations, qui sont envoyées par le dispositif de communication (3),
**caractérisé en ce que**
le dispositif de communication (3) est aménagé pour contrôler si le signal d'identification est le signal d'identification d'un employé de maintenance, et si le signal d'identification est le signal d'identification d'un employé de maintenance, et si un besoin de maintenance a été constaté par l'appareil médical (2), envoyer une alarme correspondant au besoin de maintenance ou une information correspondant au besoin de maintenance au dispositif d'identification (4).

2. Système de traitement (1) selon la revendication 1, **caractérisé en ce que** le dispositif d'identification (4) est réalisé sous la forme d'une montre, d'un téléavertisseur, d'un téléphone portable, ou d'un ordinateur portatif.

3. Système de traitement (1) selon l'une quelconque des revendications précédentes, **caractérisé en ce que** le dispositif de communication (3) de l'appareil médical (2) et le dispositif d'identification (4) sont aménagés pour communiquer l'un avec l'autre par le biais d'un standard radio commun et/ou **en ce que** le dispositif de communication (3) de l'appareil médical (2) et le dispositif d'identification (4) sont aménagés pour communiquer l'un avec l'autre par le biais d'un signal infrarouge.

4. Système de traitement (1) selon l'une quelconque des revendications précédentes, **caractérisé en ce qu'**une base de données de consignation (26) est prévue et l'appareil médical (2) est aménagé pour consigner les accès d'utilisation et les identités des utilisateurs effectuant les accès d'utilisation dans la base de données de consignation (26).

5. Système de traitement (1) selon l'une quelconque des revendications précédentes, **caractérisé en ce que** l'appareil médical (2) est un appareil de dialyse, lequel est aménagé pour la réalisation d'un traitement de dialyse.

6. Appareil médical (2) pour un système de traitement (1), lequel présente un dispositif de communication (3), lequel est aménagé pour émettre sans fil un signal d'interrogation et recevoir sans fil un signal d'identification d'un dispositif d'identification (4) porté par un utilisateur (100),
**caractérisé en ce que**
le dispositif de communication (3) est aménagé pour contrôler si le signal d'identification est le signal d'identification d'un employé de maintenance, et si le signal d'identification est le signal d'identification d'un employé de maintenance, et si un besoin de maintenance a été constaté par l'appareil médical (2), envoyer une alarme correspondant au besoin de maintenance ou une information correspondant au besoin de maintenance au dispositif d'identification (4).

7. Procédé d'identification d'un employé de maintenance (100) d'un appareil médical (2) pour un système de traitement (1) selon l'une quelconque des revendications 1 à 4, comprenant les étapes de :
- émission sans fil d'un signal d'interrogation par un dispositif de communication (3) de l'appareil médical (2) à au moins un dispositif d'identification (4) porté par un employé de maintenance (100) et identifiant celui-ci ;
- réception d'un signal d'identification émis sans fil en réponse au signal d'interrogation reçu dans le dispositif de communication (3) de l'appareil médical (2) ;
- identification de l'employé de maintenance (100) sur la base du signal d'identification reçu,
- dans lequel le dispositif de communication (3) est aménagé pour envoyer une alarme ou une information à l'employé de maintenance (100), si un besoin de maintenance a été constaté par l'appareil médical (2).

8. Procédé selon la revendication 7, **caractérisé en ce que** l'appareil médical (2) est un appareil de dialyse, lequel est aménagé pour la réalisation d'un traitement de dialyse.
